(19) Europäisches Patentamt
European Patent Office
Office européen des brevets

(11) EP 2 292 283 A1

(12) EUROPEAN PATENT APPLICATION

(43) Date of publication:
09.03.2011 Bulletin 2011/10

(51) Int Cl.:
*A61M 1/16* (2006.01)    *A61B 10/00* (2006.01)

(21) Application number: 09011352.3

(22) Date of filing: 04.09.2009

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA RS

(71) Applicant: B. Braun Avitum AG
34212 Melsungen (DE)

(72) Inventors:
• Castellarnau, Alex
34212 Melsungen (DE)
• Schreiber, Christian
34292 Ahnatal (DE)

(54) **Method and device to obtain physiological parameters and detect clinical or subclinical abnormalities during a kidney substitution treatment**

(57) The invention relates to devices and methods for detecting clinical and subclinical abnormalities during a kidney substitution treatment, by measuring continuously a kidney substitution treatment dialysate. The detection of the abnormalities is achieved by fitting the time dependency of a first set of measurements to a physiological model, extracting the physiologically relevant parameters, and comparing further measurements performed in real-time to the corresponding model predictions. Detection of a deviation from the expected behaviour triggers an alarm and/or measures to confirm or correct the abnormality detection. Estimation the rebound effects taking place after the kidney substitution treatment is also contemplated.

figure 1

EP 2 292 283 A1

**Description**

The invention relates to a method and a device to obtain physiological parameters by modelling absorbance data coming from a spectrophotometric sensor coupled with the water system of a machine able to perform kidney substitution treatments. The obtained parameters can not only be used to estimate the post-dialysis rebound, and by extension the real or equilibrated Kt/V parameter, but also for characterizing hemodynamic singularities of the patient. Additionally, evaluating deviations from the modelled or expected data is used to detect clinical or subclinical abnormalities during a kidney substitution treatment.

Dialysis adequacy is the topic that has got and gets more attention when one thinks about patient outcome. In order to estimate dialysis adequacy one needs a parameter establishing a relation between dialysis dosage and patient outcome. The most accepted parameter to estimate the quantity of dialysis delivered or dosage is the Kt/V, where K is the effective clearance for urea, t is the treatment time and V is the urea distribution volume which matches the total body water.

The NCDS (National Cooperative Dialysis Study) and the HEMO study found, after analyzing a large patient group, that morbidity and mortality in end stage renal disease (ESRD) was strongly correlated with the Kt/V value or dialysis dose. Data obtained from these studies resulted in guidelines regarding hemodialysis treatments, which demand a minimum dose of Kt/V-1.2 for non-diabetic patients and 1.4 for diabetics (DOQI guidelines). It is worth to point out that a morbidity decrease not only improves the patient well-being, but also reduces significantly the medical costs as the patient requires less care.

The need for a reliable and cost effective method to monitor the Kt/V and by extension control dialysis adequacy and morbidity is therefore easily understood.

In the Kt/V calculation, the main problems are the K and V estimation along with the multi-compartment urea kinetics. V can be estimated by bioimpedance, anthropometric measurements or applying the urea kinetic model (UKM). All these methods have a certain degree of error. K can be estimated so far by measuring the blood urea concentration before and after the treatment or by monitoring inlet and outlet conductivity changes in the dialysate side.

The method of taking blood samples is used as a reference. After taking the blood samples and applying either the UKM or the Daugirdas formula a single pool Kt/V (spKt/V) is estimated. Furthermore, Daugirdas second generation formulas should be used to get an equilibrated Kt/V (eKt/V) which accounts for the urea rebound caused by the fact that urea kinetics do not follow a single pool model but a multi-compartment one. This method has two main problems:

-    It is not possible to know whether the treatment is adequate or not before it finishes, therefore it is not possible to perform any action to improve the situation
-    It is not a method which is easy to apply: sampling time is very important to get an accurate value, and the medical staff must send the samples to the lab, wait for the results and calculate Kt/V values with the help of a computer.

These facts result in monthly basis Kt/V measurements in the best case, which means that in a worst case scenario a patient might be under-dialyzed for one whole month.

Conductivity methods are based on the observation that sodium clearance is almost equal to urea clearance and that the relationship between dialysate conductivity and dialysate sodium concentration can be considered linear in the temperature range of interest. Therefore it is possible to get urea clearance by measuring the sodium diffusion transport through the membrane in the dialyzer.

It is important to introduce the concept of Dialysance, as it slightly differs from Clearance:

Clearance is defined as the ratio between transport rate and concentration multiplied by flow, and it is applicable when the diffusing substance is on the blood side but not on the dialysate, as is the case for urea.

Dialysance is defined as the ratio between transport rate and concentration gradient multiplied by flow, and it is applicable when the diffusing substance is in both dialyzer sides. When one applies conductivity methods to measure urea Clearance, one actually measures sodium Dialysance (Depner T, Garred L., Solute transport mechanisms in dialysis. Hörl W, Koch K, Lindsay R, Ronco C, Winchester JF, editors. Replacement of renal function by dialysis, 5th ed. Kluwer academic publishers, 2004:73-91).

During conductivity based clearance measurements, a dialysate inlet conductivity different to the blood one is produced, which results in a net transfer of sodium either from blood to dialysate or from dialysate to blood due to the generated gradient. There are currently several methods which are applied in the industry:

In a first method a one-step conductivity profile is performed; in a second method a two-step conductivity profile is performed; and in a third method an integration of conductivity peaks is used (Polaschegg HD, Lcvin NW. Hemodialysis machines and monitoris. Hörl W, Koch K, Lindsay R, Ronco C, Winchester JF, editors. Replacement of renal function by dialysis, 5th ed. Kluwer academic publishers, 2004:414-418). The main advantage of this approach is that it is relatively easy to implement and cost effective as it only needs an extra conductivity/temperature sensor downstream the dialyzer. It offers Kt/V measurements during the treatment allowing the medical staff to react and perform some actions in case the treatment is not going as it should. However, conductivity based methods have also some limitations: they can induce some sodium load in the patient during the measurement; and they arc not useful to obtain other interesting parameters like nPCR or TRU. The maximum measurement frequency offered so far by the industry is about 20 minutes, which means that in a worst case scenario the patient could be under-dialyzed for 20 minutes. And although there are some publications claiming it, so far, conductivity methods haven't been applied with enough reliability to hemofiltration or hemodiafiltration treatments.

Another method to estimate hemodialysis adequacy is by direct measurement of the waste products (urea) concentration in the effluent dialysate. This method assumes that the evolution of urea concentration over the time in the dialysate side is proportional to the one in the blood, therefore the slope of the line obtained after applying the natural logarithm to the registered concentration values over the time will be the same on both sides: dialysate side and blood side. And by definition such a slope is K/V, which multiplied by the therapy time results in the Kt/V value.

There are two different methods available to measure online the concentration of waste products in effluent dialysate: Urea sensors and UV spectrophotometry.

The limitations of the urea sensors are well known. Recent works carried out by Fridolin 1. et al (I. Fridolin, M. Magnusson, L.-G. Lindberg. On-line monitoring of solutes in dialysate using absorption of ultraviolet radiation: Technique description. The International Journal of Artificial Organs. Vol, 25, no. 8,2002, pp. 748-761) and Uhlin F. (Uhlin F. Haemodialysis treatment monitored online by ultra violet absorbance. Linköping University Medical Dissertations n° 962. Department of Medicine and Care Division of Nursing Science & Department of Biomedical Engineering. 2006.) have shown that UV spectrophotometry is a reliable and cost affordable method to monitor waste products in effluent dialysate. Additionally, the European Patent EP183948BI describes a sensor coupled with the dialysate flow system of a dialysis machine, which is actually an UV spectrophotometer measuring UV absorbance of UV absorbing products in spent dialysate.

Years ago research effort aimed to model the urea kinetics was performed. This resulted in a compartment based urea kinetic model, where it was assumed that the human body was divided in intracellular and extracellular pools. Later a more physiological model based on body spaces and perfusion flow was developed (Dedrick RL, Gabelnick HL, Bischoff BK. Kineties of Urea Distribution. Proc Ann Conf Engr Med Biol 1968; 10:36-41). During the carly nineties, Schneditz and Daugirdas further developed the perfusion model and published a formal analytical solution for it. (Schneditz I), Daugirdas JT. Formal analytical solution to a regional blood flow and diffusion based urea kinetic model. ASAIO J. 1994 Jul-Sep;40(3):M667-73).

The mentioned models describe the blood urea concentration during a dialysis treatment and the subsequent urea rebound when the patient is disconnected from the machine. If the treatment conditions are kept constant or within small variations, the concentration of solutes in spent dialysate differs from the one in blood by a constant offset. The availabilily of spectrophotometric sensors, which continuously assess the decay of concentration of uremic compounds in spent dialysate, allows real-time kinetic modelling by fitting the above mentioned models on the measured data. A proper fitting procedure could deliver good estimations of the physiological parameters playing an important role during a therapy, i.e. cardiac output, blood access flow, urea generation, residual kidney function, dialysis clearance and volume and flow fractions of the body. During the post-dialysis stage the obtained parameters keep on governing the body distribution of solutes; therefore they can be used to calculate the rebound effect and by extension an equilibrated Kt/V with enough accuracy.

Because the physiological parameters tend to be constant for a given patient, it is possible to store them in a suitable media, i.e. a patient card. The stored parameters can be used to estimate the concentration or absorbance decay of waste products in spent dialysate. Any deviation from the modelled data can be interpreted as a therapy anomaly, for example a subclinical hypotension. Said anomaly can be accordingly controlled, for example by triggering a blood pressure measurement or decreasing the ultrafiltration rate.

Patent EP0847768B1 discloses a method and a device to estimate the Kt/V and other dialysis adequacy parameters by analyzing the urea concentration decay in spent dialysate. By this method, however, the measuring device samples the spent dialysate instead of being directly coupled with the dialysate flow; and the fitting procedure is not based on a physiological approach but on experimental exponential curves.

The problem of this invention is to provide a reliable method and device to estimate physiological parameters based on concentration or absorbance data delivered by a measuring cell coupled with the water system of a dialysis machine; to use the modelled parameters to estimate the rebound effects and the equilibrated Kt/V; to detect anomalies during the therapy based on the modelled data; and, if necessary, to take measures against the characterized anomalies. This problem is solved by a method and device with the features described in claim 1. Furthermore this problem is solved by a device with the features described in claim 17. Preferred embodiments of the invention arc described in the claims 1 to 17.

Further goals, advantages, features and possibilities of use of this invention arise out of the subsequent description of the embodiments of the invention. Therefore every described or depicted feature of its own or in arbitrary meaningful combination forms the subject matter of the invention even independent of its summary in the claims or its reference to other claims.

It shows:

FIG I    Depicts a portion of a conventional dialysis machine plus a slight modification to host a sensor coupled with the dialysate circuit,

FIG 2    Depicts an schema of the regional blood flow model formally solved by Schneditz and Daugirdas (Formal analytical solution to a regional blood flow and diffusion based urea kinetic model. ASAIO J. 1994 Jul-Scp;40 (3):M667-73).

FIG 3    Depicts a fit of real UV-absorbance data, continuously measured in spent dialysate, using the formal solution of the regional blood flow model. The fitted physiological parameters for this 86 years old male patient are CO: 4.09 l/min; $fQ_{HFS}$: 0.81; $fV_{HFS}$: 0.21; TBW: 28.74 1; $A(0)_{HfS/LFS}$: 1.04 a.u.; G: 0.019 l/min; $Q_{AC}$: 0.22 l/min.

FIG 4    Depicts a fit of real UV-absorbance data, continuously measured in spent dialysate, by using the formal solution of the regional blood flow model algorithm together with an extended segmentation algorithm, which improves the goodness of the fit.

FIG 5    Discloses a regional blood How model with three body pools: a splanchnic (fast perfusion) pool with volume EF. a somatic (slow perfusion) pool with volume ES and a blood pool with volume EB. There is a clearance KS between the blood and somatic pools, a clearance KF between the blood and the splanchnic pools, and a residual renal clearance KS and a dialysis clearance KD describing the removal of substances out of the blood compartment.

FIG 6    Shows a real data graphical solution of the tri-exponential model.

FIG 7    Shows a deviation of the actual measured curve when compared with the modelled curve. The observed bump after 60 minutes of treatment can be explained by a subclinical hypotensive episode.

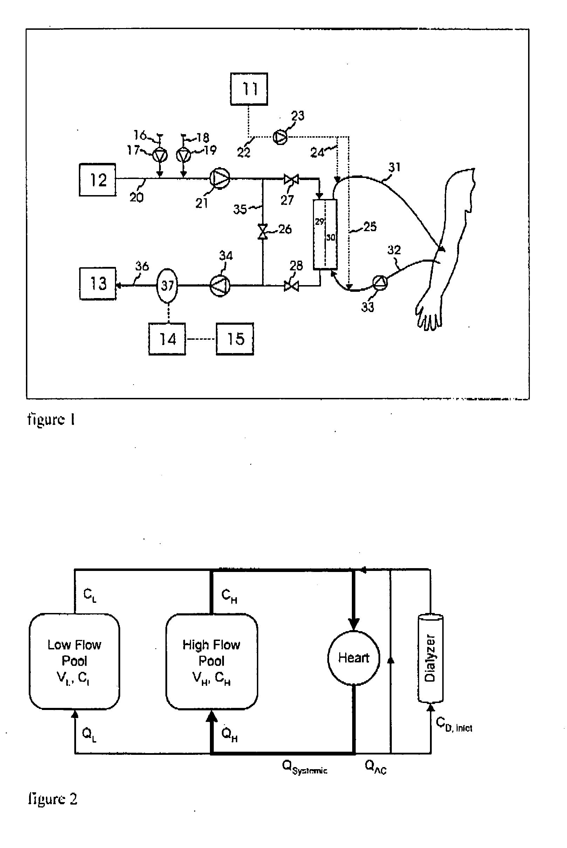

FIG. 1 shows a sketch of the dialysate circuit of a conventional dialysis machine plus a slight modification to host a sensor coupled with the dialysate circuit. The blood from a patient is taken out into an extracorporeal circuit, it flows through the tube 32 into the blood chamber 30 of a dialyzer and returns to the patient through the tube 31. The flow rate of the blood circuit is controlled by the blood pump 33. The dialysis fluid is made of several concentrates and water, therefore the machine disclosed in figure 1 comprises a water inlet 12, two concentrates inlets 16 and 18 and two concentrate pumps 17 and 19. The water flow together with the concentrates flow defines the final properties of the dialysis fluid. The conduit 20 takes the dialysis fluid to the dialysate chamber 29 of the dialyzer, which is separated from the blood chamber 30 by a semi permeable membrane. The dialysis fluid it is pumped into the dialyzer by the pump 21. A second pump 34 sucks the dialysis fluid and any ultrafiltrate removed from the blood. A bypass line 35 is arranged between the pumps 21 and 34. Several valves 26, 27 and 28 are arranged to control the dialysate flow. The conduit 36 leads the spent dialysate to a UV-sensor 37 measuring its light absorbance, the UV-sensor 37 is connected by an interface with the computer 14 which processes the measured data, the result of the data processing is displayed and/or printed by the device 15, which is connected with the computer 14 by an interface. The conduit 36 leads the spent dialysate after its measurement by the UV-sensor 37 to the drain system 13. The dotted lines 22, 24 and 25 represent an adaptation of the disclosed apparatus for hemodiafiltration treatments. The substitution fluid comes from a substitution fluid source 11, flows through the line 22 and is pumped in the blood lines of the patient by the pump 23. In case of post dilution hemodiafiltration the conduit 24 leads the substitution fluid to the venous line of the extracorporeal blood system; in case of pre dilution hemodiafiltration the conduit 25 leads the substitution fluid to the arterial line of she extracorporeal blood system; and in case of pre-post dilution hemodiafiltration both conduits 24 and 25 are used. The computer 14 controls all the elements shown on the figure by means of proper interfaces, said interfaces are not drawn for the sake of simplicity. The computer 14 gathers information about other parameters of the dialysis machine, i.e. blood flow, dialysate flow and/or therapy time, these parameters together with the measured data are processed, the result tunes the Kt/V measuring functionality to assess deviations.

The UV-sensor 37 can be substituted by an urea-sensor, in this case will the urea concentration in spent dialysate measured instead of the light absorbance. The disclosed dialysis machine is provided with several other means as is conventional. These other means are not disclosed, since they are not relevant for the operation of the present invention.

*Implementation of the Regional Blood Flow model (RBF)*

Fig 2 depicts the regional blood flow model solved by Schneditz and Daugirdas. The body is divided in two systems: the high flow system (HFS), which contains highly perfused organs like the heart, brain, lungs, portal system and blood; and the low flow system (LFS), which contains low perfused organs like the muscles, bones, skin and fat. The high flow system contains about 21% of the total body water (TBW) but it gets about 73% of the cardiac output in patients with end-stage renal disease (ESRD). The low flow systems contains 79% of the TBW but it gets 27% of the cardiac output.

Equations 1 and 2 describe the urea concentration in both, HFS and LFS. during dialysis and between dialysis time. The concentration terms may be substituted by absorbance terms.

$$c(t)_{HFS} = c(0)_{HFS}\frac{p_1 v^{\lambda_1} - q_1 v^{\lambda_2}}{p_1 - q_1} + c(0)_{LFS}\frac{p_1 q_1 (v^{\lambda_2} - v^{\lambda_1})}{p_1 - q_1} + k\frac{p_1\left(\frac{v^{\lambda_1}-1}{\lambda_1}\right) - q_1\left(\frac{v^{\lambda_2}-1}{\lambda_2}\right)}{p_1 - q_1} \quad (1)$$

$$c(t)_{LFS} = c(0)_{HFS}\frac{v^{\lambda_1} - v^{\lambda_2}}{p_1 - q_1} + c(0)_{LFS}\frac{p_1 v^{\lambda_2} - q_1 v^{\lambda_1}}{p_1 - q_1} + k\frac{\left(\frac{v^{\lambda_1}-1}{\lambda_1}\right) - \left(\frac{v^{\lambda_2}-1}{\lambda_2}\right)}{p_1 - q_1} \quad (2)$$

Note: For a full description of equations 1 and 2 see ASAIO J. 1994 Jul-Sep;40(3):M667-73.

In one of the preferred embodiments of the current invention the RBF model is implemented in the computer 14. The data measured by the sensor 37 is used as input for a fitting procedure. If the conditions of the treatment arc kept constant or within small variations the difference between blood concentration and spent dialysate absorbance is proportional; therefore it is possible to fit the RBF model with the measured UV-absorbance to obtain good estimations of the following physiological parameters:

- CO: Cardiac ouput.
- $fQ_{HFS}$: Fraction of the systemic now perfusing the HFS.
- $fV_{HFS}$: Fraction of the total body water contained by the HFS.
- TBW: Total body water.
- $A(0)_{HFS/LFS}$: Initial UV absorbance in the HFS and LFS.
- G: Urea or "absorbance" generation.
- $Q_{AC}$: Blood flow on the patient access: AV fistula or AV graft.
- $K_{AC}$: Clearance of the dialysis procedure.
- $K_R$: Residual renal function.

The obtained parameters can be used to estimate the post-dialysis rebound effects. which allows a good estimation of the eKt/V value.

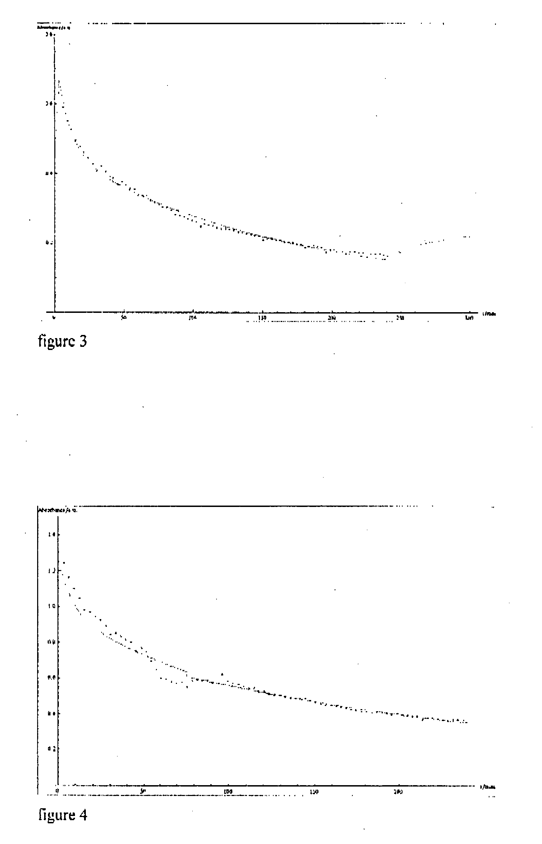

Fig 3 shows real measured UV-absorbance data fitted using the RBF. The patient was 86 years old and weighed 57 kg. The obtained physiological parameters were:

| | |
|---|---|
| CO | 4.09 l/mm |
| $fQ_{HFS}$ | 0.81 |
| $fV_{HFS}$ | 0.2 |
| TBW | 28.74 |

(continued)

| A(0)$_{HFS/LFS}$ | 1.04 a.u. |
|---|---|
| G | 0.019 l/min |
| Q$_{AC}$ 0.22 | 0.22 l/min |

An cKt/V of 1.31 was estimated with the modelled parameters. A blood based eKt/V for urea of 1.28 was calculated using an equilibrated blood sample, which was taken 30 minutes after the end of the dialysis procedure. The agreement between both values is outstanding.

Special situations during dialysis, i.e. patient position change, sport during the treatment, or subclinical hypotension, cause atypical patterns in the absorbance curves. In order to avoid poor fits in such situations a segmentation algorithm is used. The quality of the fit $\Gamma(t)$ is evaluated at each data point by means of equation 3.

$$\Gamma(t) = \left( \frac{Abs_{fit}(t) - Abs_{data}(t)}{Abs_{data}(t)} \right) \quad (3)$$

A critical quality, namely $\Gamma_c$=0.04, is defined. A time point $\tau$ such that for all times t > $\tau$ the quality of the fit $\Gamma(t)$ is better than $\Gamma_c$ is found. $\tau$ divides the data in two sections which arc individually fitted. The kinetic parameters obtained by the fit of the segment such that t > $\tau$ are used to predict the rebound effects. Fig 4 depicts the effect of the segmentation algorithm, in that case $\tau$ is about 75 minutes.

The applicants have however realized that the absorbance curves can be extremely well described by a three-exponential decay, i.e. six parameters. Solving a system of six equations with ten unknowns is known to be mathematically impossible as the system is underdetermined. This fact raised doubts about a reliable application of the RBF model using as input the UV-absorbance measured in spent dialysate, and led the applicants to the derivation of a tri-exponential regional blood flow model.

*Derivation and implementation of a tri-exponential regional blood flow model*

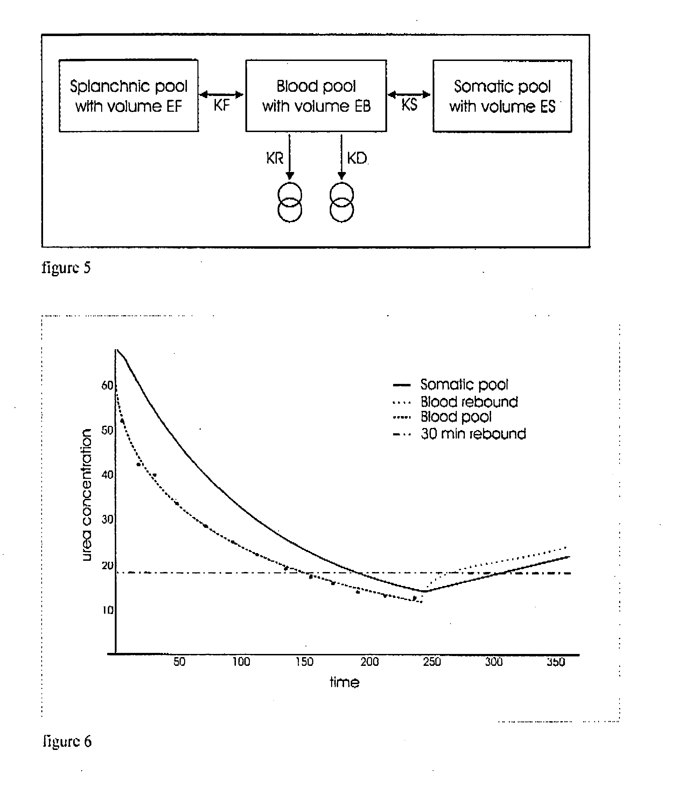

A second, and arguably much easier approach to the same problem is depicted in Fig. 5. In this model, we have a blood compartment with a blood water volume EB and a urea concentration $C_B(t)$; a somatic (slow) volume with a water volume ES and a urea concentration $C_S(t)$; and a splanchnic (fast) compartment with a water volume EF and a urea concentration $C_F(t)$. Between the blood and the somatic volume, there is a clearance KS; between the splanchnic and the blood volume, there is a clearance KF; and within the blood space there is a KR, which is the residual renal function, and a KD which is the clearance due to the dialysis procedure.

The mathematical derivation of the model requires a separation between the dialysis procedure, where KD is present, and the post-dialysis rebound stage, where KD is absent and a the equilibration of concentrations takes place.

*1. Model derivation during the dialysis treatment*

Using mass conservation, we obtain during a dialysis treatment:

$$\partial m_F = (C_B - C_F) \cdot KF \cdot \partial t \quad (4)$$

$$\partial m_S = (C_B - C_S) \cdot KS \cdot \partial t \quad (5)$$

$$\partial m_B = (-\partial m_F - \partial m_S - C_B \cdot KR - C_B \cdot KD) \cdot \partial t \quad (6)$$

Dividing by the volumes of each compartment allows to express the differential equations in terms of concentration:

$$\partial C_F = \frac{(C_B - C_F)}{EF} \cdot KF \cdot \partial t \qquad (7)$$

$$\partial C_S = \frac{(C_B - C_S)}{ES} \cdot KS \cdot \partial t \qquad (8)$$

$$\partial C_B = -\frac{1}{EB}\left((C_B - C_F) \cdot KF + (C_B - C_S) \cdot KS + C_B \cdot KR + C_B \cdot KD\right) \cdot \partial t \qquad (9)$$

Expressing equations 7, 8 and 9 in matrix notation:

$$\frac{\partial}{\partial t}\begin{pmatrix} C_B(t) \\ C_F(t) \\ C_S(t) \end{pmatrix} = \begin{pmatrix} -\dfrac{KS + KF + KR + KD}{EB} & \dfrac{KF}{EB} & \dfrac{KS}{EB} \\ \dfrac{KF}{EF} & -\dfrac{KF}{EF} & 0 \\ \dfrac{KS}{ES} & 0 & -\dfrac{KS}{ES} \end{pmatrix} \cdot \begin{pmatrix} C_B(t) \\ C_F(t) \\ C_S(t) \end{pmatrix} \qquad (10)$$

where we denote the matrix by **M**. Solving this equation is non-trivial as it involves solving a cubic equation. However, we can use our knowledge of the system to solve it. One key feature of an equation of this type is that any solution $C_{R/F/S}$ (t) of any of the concentrations will follow a tri-exponential form. Thus, it is possible to write the following solved model:

$$C_B(t) = A_B e^{\lambda_1 t} + B_B e^{\lambda_2 t} + C_B e^{\lambda_3 t} \qquad (11)$$

$$C_F(t) = A_F e^{\lambda_1 t} + B_F e^{\lambda_2 t} + C_F e^{\lambda_3 t} \qquad (12)$$

$$C_S(t) = A_S e^{\lambda_1 t} + B_S e^{\lambda_2 t} + C_S e^{\lambda_3 t} \qquad (13)$$

where:

- $\lambda_{1/2/3}$ are the eigenvalues of M, equal among pools.
- Coefficients $A_{B/F/S}$, $B_{R/F/S}$ and $C_{B/F/S}$ arc different among pools.
- Coefficients $A_B$, $B_B$, $C_B$, $\lambda_1$, $\lambda_2$ and $\lambda_3$ are known because of fitting either the blood pool data or the spent dialysate data.

It follows from equations 7 and 8; and the assumption that at the beginning of the dialysis treatment the three pools are equilibrated: $C_B = C_F = C_S$; the boundary condition such that for t-0:

$$\frac{\partial C_F}{\partial t} = 0 \text{ and } \frac{\partial C_S}{\partial t} = 0$$

It is then possible to derive the coefficients A, B and C for the fast and slow pools from the known coefficients.

Let $\alpha_F = \dfrac{KF}{EF}$ and $\alpha_S = \dfrac{KS}{ES}$, it is then possible to rewrite equations 7 and 8 as:

$$\dot{C}_F(t) = \alpha_F \cdot \left( C_B(t) - C_F(t) \right) \qquad (14)$$

$$\dot{C}_S(t) = \alpha_S \cdot \left( C_B(t) - C_S(t) \right) \qquad (15)$$

By substituting the values $C_B(t)$, $C_F(t)$ and $C_S(t)$ on equations 14 and 15 by the expressions 11, 12 and 13, it follows:

$$C_F(t) = \alpha_F \cdot \left( \left( A_B e^{\lambda_1 t} + B_B e^{\lambda_2 t} + C_B e^{\lambda_3 t} \right) - \left( A_F e^{\lambda_1 t} + B_F e^{\lambda_2 t} + C_F e^{\lambda_3 t} \right) \right) \quad (16)$$

$$C_S(t) = \alpha_S \cdot \left( \left( A_B e^{\lambda_1 t} + B_B e^{\lambda_2 t} + C_B e^{\lambda_3 t} \right) - \left( A_S e^{\lambda_1 t} + B_S e^{\lambda_2 t} + C_S e^{\lambda_3 t} \right) \right) \quad (17)$$

Refactoring equations 16 and 17 yields:

$$\dot{C}_F(t) = \alpha_F \cdot \left( A_B - A_F \right) \cdot e^{\lambda_1 t} + \alpha_F \cdot \left( B_B - B_F \right) \cdot e^{\lambda_2 t} + \alpha_F \cdot \left( C_B - C_F \right) \cdot e^{\lambda_3 t} \quad (18)$$

$$\dot{C}_S(t) = \alpha_S \cdot \left( A_B - A_S \right) \cdot e^{\lambda_1 t} + \alpha_S \cdot \left( B_B - B_S \right) \cdot e^{\lambda_2 t} + \alpha_S \cdot \left( C_B - C_S \right) \cdot e^{\lambda_3 t} \quad (19)$$

Differentiating equations 12 and 13 yields:

$$\dot{C}_F(t) = \lambda_1 A_F \cdot e^{\lambda_1 t} + \lambda_2 B_F \cdot e^{\lambda_2 t} + \lambda_3 C_F \cdot e^{\lambda_3 t} \quad (20)$$

$$\dot{C}_S(t) = \lambda_1 A_S \cdot e^{\lambda_1 t} + \lambda_2 B_S \cdot e^{\lambda_2 t} + \lambda_3 C_S \cdot e^{\lambda_3 t} \quad (21)$$

The exponential coefficients of equations 18 and 19 must equal the ones of equations 20 and 21. Thus, for the fast flow pool it is possible to write:

$$\lambda_1 A_F = \alpha_F \cdot (A_B - A_F) \Leftrightarrow A_F = \frac{\alpha_F}{\alpha_F + \lambda_1} \cdot A_B \quad (22)$$

$$\lambda_2 B_F = \alpha_F \cdot (B_B - B_F) \Leftrightarrow B_F = \frac{\alpha_F}{\alpha_F + \lambda_2} \cdot B_B \quad (23)$$

$$\lambda_3 C_F = \alpha_F \cdot (C_B - C_F) \Leftrightarrow C_F = \frac{\alpha_F}{\alpha_F + \lambda_3} \cdot C_B \quad (24)$$

and for the low flow pool:

$$\lambda_1 A_S = \alpha_S \cdot (A_B - A_S) \Leftrightarrow A_S = \frac{\alpha_S}{\alpha_S + \lambda_1} \cdot A_B \quad (25)$$

$$\lambda_2 B_S = \alpha_S \cdot (B_B - B_S) \Leftrightarrow B_S = \frac{\alpha_S}{\alpha_S + \lambda_2} \cdot B_B \quad (26)$$

$$\lambda_3 C_S = \alpha_S \cdot (C_B - C_S) \Leftrightarrow C_S = \frac{\alpha_S}{\alpha_S + \lambda_3} \cdot C_B \quad (27)$$

because at t=0, $\dfrac{\partial C_F}{\partial t} = 0$ and $\dfrac{\partial C_S}{\partial t} = 0$, from equations 20 and 21 follows:

$$0 = \lambda_1 A_F + \lambda_2 B_F + \lambda_3 C_F \quad (28)$$

$$0 = \lambda_1 A_S + \lambda_2 B_S + \lambda_3 C_S \quad (29)$$

where the coefficients $A_{F/S}$, $B_{F/S}$ and $C_{F/S}$ can be substituted by the expressions 22 to 27. $\alpha_F$ and $\alpha_S$ can be then solved. Further, by knowing $\alpha_F$ and as, $A_{F/S}$, $B_{F/S}$ and $C_{F/S}$ can be also solved. At this point the solutions 11, 12 and 13 of the model are known. Moreover, by knowing $\alpha_F$ and $\alpha_S$, the rows 2 and 3 of the matrix M arc also known.

The first row of the matrix M can be also derived by using the already obtained coefficients:

$$\text{let } M(1,1) = \alpha = -\frac{KS + KF + KR + KD}{EB} \; ; \; M(1,2) = \beta = \frac{KF}{EB} \; ; \text{ and } M(1,3) = \gamma = \frac{KS}{EB} \; .$$

from M follows:

$$C_B(t) = \alpha \cdot C_B(t) + \beta \cdot C_F(t) + \gamma \cdot C_S(t) \quad (30)$$

using expressions 11, 12, and 13 to substitute the factors $C_B(t)$ $C_F(t)$ and $C_S(t)$ on equation 30 yields:

$$C_B(t) = \alpha \cdot \left(A_B e^{\lambda_1 t} + B_B e^{\lambda_2 t} + C_B e^{\lambda_3 t}\right) + \beta \cdot \left(A_F e^{\lambda_1 t} + B_F e^{\lambda_2 t} + C_F e^{\lambda_3 t}\right) + \gamma \cdot \left(A_S e^{\lambda_1 t} + B_S e^{\lambda_2 t} + C_S e^{\lambda_3 t}\right) \quad (31)$$

refactoring equation 31 yields:

$$C_B(t) = \left(\alpha \cdot A_B + \beta \cdot A_F + \gamma \cdot A_S\right) \cdot e^{\lambda_1 t} + \left(\alpha \cdot B_B + \beta \cdot B_F + \gamma \cdot B_S\right) \cdot e^{\lambda_2 t} + \left(\alpha \cdot C_B + \beta \cdot C_F + \gamma \cdot C_S\right) \cdot e^{\lambda_3 t} \quad (32)$$

differentiating equations 11 yields:

$$C_B(t) = \lambda_1 A_B \cdot e^{\lambda_1 t} + \lambda_2 B_B \cdot e^{\lambda_2 t} + \lambda_3 C_B \cdot e^{\lambda_3 t} \quad (33)$$

the exponential coefficients of equations 32 and 33 must be equal. Thus, it is possible to write a system of three equations and solve for the three unknowns $\alpha$, $\beta$ and $\gamma$:

$$\left. \begin{aligned} \alpha \cdot A_B + \beta \cdot A_F + \gamma \cdot A_S &= \lambda_1 \cdot A_B \\ \alpha \cdot B_B + \beta \cdot B_F + \gamma \cdot B_S &= \lambda_2 \cdot B_B \\ \alpha \cdot C_B + \beta \cdot C_F + \gamma \cdot C_S &= \lambda_3 \cdot C_B \end{aligned} \right\} \quad (34)$$

*2. Derivation of the model volume fractions: $fV_B$. $fV_F$ and $fV_S$*

By using the solved coefficients of the matrix M it is possible to derive the body volume fractions of each of the modelled regions, namely the blood, splanchnic and somatic compartments.
$fV_B$ can be expressed as EB divided by TBW (total body water). Thus, it is possible to write:

$$fV_B = \frac{EB}{TBW} = \frac{EB}{EB + EF + ES} = \frac{EB/EB}{EB/EB + EF/EB + ES/EB} \quad (35)$$

$$fV_B = \frac{1}{1 + EF/EB + ES/EB} \quad (36)$$

from the factors EF/EB and ES/EB of equation 36, it can be derived:

$$\frac{EF}{EB} = \frac{KF}{EB} \cdot \frac{ES}{KF} = \frac{KF}{EB} \cdot \frac{1}{\alpha_F} \quad (37)$$

$$\frac{ES}{EB} = \frac{KS}{EB} \cdot \frac{ES}{KS} = \frac{KS}{EB} \cdot \frac{1}{\alpha_S} \quad (38)$$

thus:

$$fV_B = \frac{1}{1 + \dfrac{EF/EB}{\alpha_F} + \dfrac{ES/EB}{\alpha_S}} \quad (39)$$

$fV_F$ can be expressed as EF divided by TBW (total body water). Thus, it is possible to write:

$$fV_F = \frac{EF}{TBW} = \frac{EB \cdot EF/EB}{TBW} = \frac{EB}{TBW} \cdot \frac{EF}{EB} = fV_B \cdot \frac{EF}{EB} \quad (40)$$

from using equation 37 in equation 40 it follows:

$$fV_F = fV_B \cdot \frac{KF}{EB} \cdot \frac{1}{\alpha_F} \quad (41)$$

Finally $IV_S$ can be expressed as:

$$fV_S = 1 - fV_B - fV_S \quad (42)$$

*3. Model derivation during the post-dialysis rebound*

If we assume that the patient's kinetic parameters stay constant after the treatment, and that KR=0, it is possible to model and solve the post-dialysis rebound. The matrix M can be rewritten as M', where all the elements are known. Because during the rebound the dialysis treatment is over, the factor KD is absent in the matrix M'.

$$\frac{\partial}{\partial t}\begin{pmatrix} C_B''(t) \\ C_F''(t) \\ C_S''(t) \end{pmatrix} = \begin{pmatrix} -\dfrac{KS+KF}{EB} & \dfrac{KF}{EB} & \dfrac{KS}{EB} \\ \dfrac{KF}{EF} & -\dfrac{KF}{EF} & 0 \\ \dfrac{KS}{ES} & 0 & -\dfrac{KS}{ES} \end{pmatrix} \cdot \begin{pmatrix} C_B''(t) \\ C_F''(t) \\ C_S''(t) \end{pmatrix} \qquad (43)$$

The solution to the system of differential equations 43 is of the form:

$$C_B''(t) = A_B' e^{\lambda_1' t} + B_B' e^{\lambda_2' t} + C_B' e^{\lambda_3' t} \qquad (44)$$

$$C_F''(t) = A_F' e^{\lambda_1' t} + B_F' e^{\lambda_2' t} + C_F' e^{\lambda_3' t} \qquad (45)$$

$$C_S''(t) = A_S' e^{\lambda_1' t} + B_S' e^{\lambda_2' t} + C_S' e^{\lambda_3' t} \qquad (46)$$

where $\lambda_1'$, $\lambda_2'$ and $\lambda_3'$ are the eigenvalues of M', and can be solved as follows:

$$\underline{M'} \cdot e = \lambda \cdot e = \lambda \cdot I \cdot e \qquad (47)$$

$$(\underline{M'} - \lambda \cdot I) \cdot e = 0 \Leftrightarrow \det(\underline{M'} - \lambda \cdot \underline{I}) = 0 \qquad (48)$$

$$\begin{vmatrix} -\dfrac{KS+KF}{EB} - \lambda' & \dfrac{KF}{EB} & \dfrac{KS}{EB} \\ \dfrac{KF}{EF} & -\dfrac{KF}{EF} - \lambda' & 0 \\ \dfrac{KS}{ES} & 0 & -\dfrac{KS}{ES} - \lambda' \end{vmatrix} \qquad (49)$$

$$\lambda_1 = \frac{-EF \cdot ES \cdot (KF + KS) - EB \cdot (ES \cdot KF + EF \cdot KS)}{2 \cdot EB \cdot EF \cdot ES} +$$

$$\frac{\sqrt{-4 \cdot EB \cdot EF \cdot ES \cdot (EB + EF + ES) \cdot KF \cdot KS + ((EB + EF) \cdot ES \cdot KF + EF \cdot (EB + ES) \cdot KS)^2}}{2 \cdot EB \cdot EF \cdot ES} \qquad (50)$$

$$\lambda_2 = \frac{-EF \cdot ES \cdot (KF + KS) - EB \cdot (ES \cdot KF + EF \cdot KS)}{2 \cdot EB \cdot EF \cdot ES} -$$

$$\frac{\sqrt{-4 \cdot EB \cdot EF \cdot ES \cdot (EB + EF + ES) \cdot KF \cdot KS + ((EB + EF) \cdot ES \cdot KF + EF \cdot (EB + ES) \cdot KS)^2}}{2 \cdot EB \cdot EF \cdot ES} \quad (51)$$

$$\lambda_3 = 0 \quad (52)$$

The third eigenvalue corresponds to a constant offset, which agrees with the physical understanding of the system, as it will reach the equilibrium at some time point.

To further solve the model it is necessary to refactor the coefficients $A'_{B/F/S}$, $B'_{B/F/S}$ and $C'_{B/F/S}$ in a Fashion similar to the one followed above.

From the matrix M' it follows:

$$C'_F(t) = \frac{KF}{EF} \cdot C''_B(t) - \frac{KF}{EF} \cdot C''_F(t) + 0 \cdot C'_S(t) = \frac{KF}{EF} \cdot (C''_B(t) - C''_F(t)) \quad (53)$$

$$C''_S(t) = \frac{KS}{ES} \cdot C''_B(t) + 0 \cdot C''_F(t) - \frac{KS}{ES} \cdot C_S(t) = \frac{KS}{ES} \cdot (C''_B(t) - C''_S(t)) \quad (54)$$

By substituting the values $C'_B(t)$, $C'_F(t)$ and $C'_S(t)$ on equations 53 and 54 by the expressions 44, 45 and 46, it follows:

$$C_F(t) = \frac{KF}{EF} \cdot \left( \left( A'_B e^{\lambda_1 t} + B'_B e^{\lambda_2 t} + C''_B e^{\lambda_3 t} \right) - \left( A'_F e^{\lambda_1 t} + B'_F e^{\lambda_2 t} + C'_F e^{\lambda_3 t} \right) \right) \quad (55)$$

$$C_S(t) = \frac{KS}{ES} \cdot \left( \left( A'_B e^{\lambda_1 t} + B'_B e^{\lambda_2 t} + C''_B e^{\lambda_3 t} \right) - \left( A'_S e^{\lambda_1 t} + B'_S e^{\lambda_2 t} + C'_S e^{\lambda_3 t} \right) \right) \quad (56)$$

Refactoring equations 55 and 56 yields:

$$C_F(t) = \frac{KS}{ES} \cdot (A'_B - A'_F) \cdot e^{\lambda_1 t} + \frac{KS}{ES} \cdot (B'_B - B'_F) \cdot e^{\lambda_2 t} + \frac{KS}{ES} \cdot (C''_B - C''_F) \cdot e^{\lambda_3 t} \quad (57)$$

$$C_S(t) = \frac{KS}{ES} \cdot (A'_B - A'_S) \cdot e^{\lambda_1 t} + \frac{KS}{ES} \cdot (B'_B - B'_S) \cdot e^{\lambda_2 t} + \frac{KS}{ES} \cdot (C'_B - C''_S) \cdot e^{\lambda_3 t} \quad (58)$$

Differentiating equations 45 and 46 yields:

$$C_F''(t) = \lambda_1' A_F' e^{\lambda_1' t} + \lambda_2' B_F' e^{\lambda_2' t} + \lambda_3' C_F' e^{\lambda_3' t} \qquad (59)$$

$$C_S''(t) = \lambda_1' A_S' e^{\lambda_1' t} + \lambda_2' B_S' e^{\lambda_2' t} + \lambda_3' C_S' e^{\lambda_3' t} \qquad (60)$$

The exponential coefficients of equations 57 and 58 must equal the ones of equations 59 and 60. Thus, for the fast flow pool it is possible to write:

$$\lambda_1' A_F' = \frac{KS}{ES} \cdot (A_H' - A_F') \Leftrightarrow A_H' = \frac{\lambda_1' + KS/ES}{KS/ES} \cdot A_F' \qquad (61)$$

$$\lambda_2' B_F' = \frac{KS}{ES} \cdot (B_H' - B_F') \Leftrightarrow B_H' = \frac{\lambda_2' + KS/ES}{KS/ES} \cdot B_F' \qquad (62)$$

$$\lambda_3' C_F'' = \frac{KS}{ES} \cdot (C_H'' - C_F') \Leftrightarrow C_H'' = \frac{\lambda_3' + KS/ES}{KS/ES} \cdot C_F'' \qquad (63)$$

and for the low flow pool:

$$\lambda_1' A_S' = \frac{KS}{ES} \cdot (A_H' - A_S') \Leftrightarrow A_H' = \frac{\lambda_1' + KS/ES}{KS/ES} \cdot A_S' \qquad (64)$$

$$\lambda_2' B_S' = \frac{KS}{ES} \cdot (B_H' - B_S') \Leftrightarrow B_H' = \frac{\lambda_2' + KS/ES}{KS/ES} \cdot B_S' \qquad (65)$$

$$\lambda_3' C_S'' = \frac{KS}{ES} \cdot (C_H'' - C_S') \Leftrightarrow C_H'' = \frac{\lambda_3' + KS/ES}{KS/ES} \cdot C_S'' \qquad (66)$$

$\lambda_3$-0, thus,

$$C''_B = C''_F = C''_S \qquad (67)$$

A boundary condition during the post-dialysis rebound is that at t-0, the concentration of each of the pools, i.e. blood, splanchnic and somatic, equals their respective concentrations at the end of the dialysis treatment $t=T_{dial}$, which are known. Therefore, it is possible to write:

$$
\left.
\begin{aligned}
C''_B(0) &= C_B(T_{dial}) = A'_B e^{\lambda'_1 t} + B'_B e^{\lambda'_2 t} + C''_B e^{\lambda'_3 t} \\
C''_F(0) &= C_F(T_{dial}) = A'_F e^{\lambda'_1 t} + B'_F e^{\lambda'_2 t} + C''_F e^{\lambda'_3 t} \\
C''_S(0) &= C_S(T_{dial}) = A'_S e^{\lambda'_1 t} + B'_S e^{\lambda'_2 t} + C''_S e^{\lambda'_3 t}
\end{aligned}
\right\} \qquad (68)
$$

Because t=0 and expression 67, it follow from expression 68:

$$
\left.
\begin{aligned}
C'_B(0) &= C_B(T_{dial}) = A'_B + B'_B + C''_F \\
C''_F(0) &= C_F(T_{dial}) = A'_F + B'_F + C'_F \\
C''_S(0) &= C_S(T_{dial}) = A'_S + B'_S + C'_F
\end{aligned}
\right\} \qquad (69)
$$

where,

- $A'_B$ and $B'_B$ can be expressed in terms of $A'_F$ and $B'_F$ by using equations 61 and 62.
- $A'_S$ and $B'_S$ can be expressed in terms of $A'_F$ and $B'_F$ by combining equations 61 and 64: and 62 and 65. Terms $A'_B$ and $B'_B$ go and the resulting equations are solved for $A'_S$ and $B'_S$:

$$
A'_S = \frac{KS/_{ES} \cdot \left(\lambda'_1 + KF/_{EF}\right)}{KF/_{EF} \cdot \left(\lambda'_1 + KS/_{ES}\right)} \cdot A'_F \qquad (70)
$$

$$
B'_S = \frac{KS/_{ES} \cdot \left(\lambda'_2 + KF/_{EF}\right)}{KF/_{EF} \cdot \left(\lambda'_2 + KS/_{ES}\right)} \cdot B'_F \qquad (71)
$$

- When the equations system 69 is expressed in terms of $A'_F$, $B'_F$ and $C'_F$, we have three equations and three unknowns. After solving it, finding solutions for $A'_{B/S}$ and $B'_{B/S}$ using equation 61, 62, 64 and 65 is trivial.

Fig, 6 shows a graphical solution of the tri-exponential model.

*4. Estimation of the equilibration time*

During the rebound phase, and considering that $\lambda_1 \neq 0$. $\lambda_2 \neq 0$ and $\lambda_3 = 0$, we have the following functional dependence of the concentrations:

$$C_B(t) = A_n \cdot e^{\lambda_1 t} + B_n \cdot e^{\lambda_2 t} + C \qquad (72)$$

According to equation 72, the most negative $\lambda$ (i.e. the smallest one) will decay faster, so the total approach to the final constant value C is determined by the slowest decaying function, i.e. by the most positive $\lambda$. Let's assume that this particular $\lambda$ is $\lambda_2$, so for long enough times $C_B(t)$ can be approximated by a single exponential decay:

$$C_B(t) \approx B_n \cdot e^{\lambda_2 t} + C \qquad (73)$$

By comparison, in electrostatics the exponential decay of a capacitor charge Q(t) is expressed as:

$$Q(t) = Q(0) \cdot e^{-t/\tau} \qquad (74)$$

After a time t=5·$\tau$ it is assumed that the capacitor charge is completely gone.

By analogy, we can estimate the equilibration time as: $t_{eq} = 5 \cdot \dfrac{1}{\lambda_2}$

The equilibrated Kt/V can be calculated by dividing the concentration at time $t_{eq}$ by the concentration at time 0:

$$eKt/V = -\ln\left(\frac{C_B'(t = t_{eq})}{C_B(t = 0)}\right) \qquad (75)$$

*5. Validation of the tri-exponential model against real patient data*

During eight dialysis treatments the patient arterial blood was sampled every 20 minutes. The urea concentration decay over the treatment time was modelled. The urea rebound and by extension the eKt/V was calculated.
Two additional blood samples were taken from the patient: one before the start of the dialysis; and another 30 minutes after finishing the treatment, i.e. body urea concentration reached the equilibrium. By using the following equation a reference blood based cKt/V for urea was obtained.

$$eKt/V_{blood} = -\ln\left(\frac{C_{30\,min\,post-dialysis}'}{C_{pre\,dialysis}'}\right) \qquad (76)$$

The following table summarizes the results:

| Patient | Treatment | eKt/V blood | eKt/V modelled | error |
|---------|-----------|-------------|----------------|-------|
| 3 | 1 | 0.96 | 1.04 | 7% |
| 3 | 2 | 1.04 | 1.02 | -2% |
| 3 | 3 | 0.93 | 0.99 | 6% |
| 3 | 4 | 1.01 | 0.9R | -3% |
| 4 | 1 | 1.44 | 1.33 | -7% |

(continued)

| Patient | Treatment | eKt/V blood | eKt/V modelled | error |
|---------|-----------|-------------|----------------|-------|
| 4 | 2 | 1.38 | 1.31 | -5% |
| 4 | 3 | 1.47 | 1.53 | 4% |
| 4 | 4 | 1.28 | 1.32 | 3% |

Because the difference between blood concentration and spent dialysate concentration/absorbance is just an offset, the same model can be applied when continuously monitoring any uremic compound or UV-absorbance in spent dialysate.

*Storing the modelled parameters and identification of abnormalities during the treatment*

Patient based physiological parameters tend to be constant among treatments, or show patterns related with the treatment day, i.e. on Mondays patients use to come to the dialysis centre with more weight as it has been not one but two days since the last treatment.

In our preferred embodiment, the measured concentration/absorbance curve is modeled. The eKt/V and the physiological parameters arc calculated and displayed. Further, the modeled parameters arc stored in a suitable medium, i.e. a patient card or a data base in the dialysis machine. At the beginning of the next therapy the last stored parameters are loaded and used to predict the concentration or absorbance evolution over the treatment time. The following equation can be used to monitor on real-time the difference between the estimated absorbance curve and the factual measured data:

$$\theta(t) = \left( \frac{Abs_{estimated}(t) - Abs_{actual}(t)}{Abs_{actual}(t)} \right) \quad (77)$$

A critical threshold, namely $\theta_c$-0.05, is defined. If $\theta(t) > \theta_c$ a problem during the treatment, may be happening, e.g. a subclinical hypotensive period. The applicants experience shows that the following abnormalities can be recognized through the analysis of the absorbance curve:

- Clinical or subclinical hypotensive episodes: produce a bump on the curve after the first treatment hour.
- Dialyzer clotting: produce a steep drop on the absorbance curve together with an increase of the pressure difference between dialyzer inlet and outlet.
- Access recirculation: produce a steep drop on the absorbance curve.

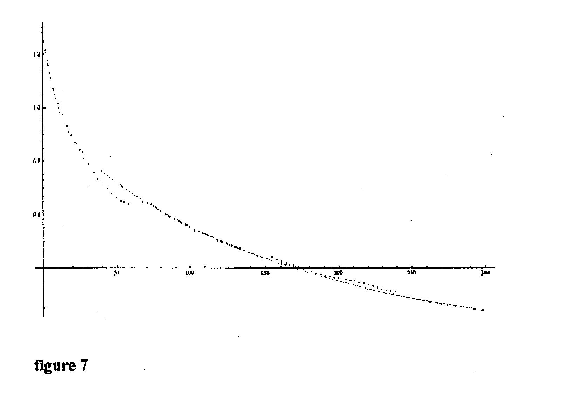

Fig. 7 shows a practical case. After 60 minutes of dialysis the actual curve shows a bump which deviates from the expected curve. This dialysis treatment was reported as normal by the medical staff. However, a blood pressure decrease together with a heart rate increase were recorded on the dialysis protocol at the same time the curve bumped. This phenomenon was caused by a too high ultrafiltration during the first treatment hour, which triggered a physiological reaction to restore the decreased volemia and blood pressure. The heart rate increase and the somatic blood supply to the central circulation raised the concentration of uremic substances on the blood compartment. Further, a UV-absorbance bolus was recorded by the UV-Sensor 37 in spent dialysate. This circumstance can be defined as a subclinical hypotensive period, as the patient didn't show any symptoms because the physiological mechanisms reacted on time. Abnormal situations requiring body regulation to keep the normal physiological status should be avoided whenever possible. In this specific case the curve bump can trigger a blood pressure measurement, and if it is below a critical level the ultrafiltration pump is stopped and a saline solution bolus might be infused to the patient.

**Claims**

1.  A method and device to obtain physiological parameters and detect clinical and subclinical abnormalities during a kidney substitution treatment,

wherein the kidney substitution treatment is provided by a machine, which has an extracorporeal blood system pumping the patient blood at a preset blood flow rate through the blood chamber of a dialyzer, which is divided by a semi-permeable membrane into the.blood chamber and a dialyzing fluid chamber, and
wherein the dialyzing fluid flows at a preset flow rate through the dialyzing fluid system of the machine and collects the waste products from the patient after flowing through the dialyzing fluid chamber of the dialyzer, and
wherein a device able to measure continuously any kidney substitution treatment related waste product to deliver together with the data provided by the kidney substitution treatment machine a adequacy parameter wherein the device is coupled with the dialyzing fluid system of the kidney substitution treatment machine, and
wherein curves describing the adequacy of the kidney substitution treatment are obtained by the device able to measure continuously any kidney substitution treatment related waste product, and
wherein there are available means to model physiological parameters and predict equilibrated adequacy parameters from the curves describing the adequacy of the kidney substitution treatment, and
wherein there are means to store the modeled physiological parameters in a suitable medium and to use them in outcoming kidney substitution treatments to estimate the evolution of the curves describing the adequacy of the kidney substitution treatment, and
wherein there are available means to identify abnormalities during the kidney substitution treatment by comparing the estimated curves describing the adequacy of the kidney substitution treatment against the actual measured curves describing the adequacy of the kidney substitution treatment, and
wherein there arc available means to trigger measures on the machine providing the kidney substitution treatment to correct or assess the identified abnormalities during the kidney substitution treatment.

2. The method and device according to claim 1, wherein the kidney substitution treatment can be double needle hemodialysis, single needle hemodialysis, single needle cross over hemodialysis, post-dilution hemodiafiltration, pre-dilution hemodiafiltration, pre-post-dilution hemodiafiltration, post-dilution hemofiltration, pre-dilution hemofiltration, pre-post-dilution hemofiltration or sequential hemodialysis.

3. The method and device according to claim 1, wherein the device able to continuously measure a waste product in dialyzing fluid is an urea sensor, an spectrophotometric sensor, and UV- spectrophotometric sensor.

4. The method and device according to claims 1 and 3, wherein the UV-spectrophotometric sensor measures in the wavelength range from 250 nm to 300 nm.

5. The method and device according to claim 1, wherein the curves describing the adequacy of the kidney substitution treatment are the timely decay of absorbance, UV-absorbance, or concentration of any uremic substance cleared during a kidney substitution treatment.

6. The method and device according to claim 1, wherein the physiological model is a regional blood flow model with a high flow system (HFS) and a low flow system (LFS), and the obtained physiological parameters are cardiac ouput; fraction of the systemic flow perfusing the high flow system; fraction of the systemic flow perfusing the low flow system; total body water, fraction of the total body water contained by the high flow system; fraction of the body water contained by the low flow system; initial absorbance, UV-absorbance or uremic compound concentration in the high flow system; initial absorbance, UV-absorbance or uremic compound concentration in the low flow system; urea generation; blood flow on the patient access; and/or clearance of the kidney substitution treatment; and wherein the equilibrated adequacy parameter is calculated by using the obtained physiological parameters.

7. The method and device according to claims 1 and 6. wherein a segmentation algorithm improve the fit agreement of the model.

8. The method and device according to claims 1, 6 and 7, wherein the segmentation algorithm defines critical quality, and a factor $\tau$, such that for all times $t > \tau$ the quality of the fit is better than the defined critical quality; and wherein said factor $\tau$ divides the data in two sections which are individually fitted

9. The method and device according to claim 1; wherein the physiological model is a tri-exponential model with one splanchnic pool with a water volume EF, one blood pool with a water volume EB and one somatic pool with a water volume ES; wherein there is a clearance KS between the blood and somatic pools, there is a clearance KF between the blood and the splanchnic pools, and there are a residual renal clearance KS and a dialysis clearance KD describing the removal of substances out of the blood compartment; and wherein the physiological parameters arc EB, EF, ES, KF, KS, KR, KD, total body water or any mathematical combination of them; and wherein the equilibrated adequacy parameter is calculated by using the obtained physiological parameters.

10. The method and device according to claims 1, 6 and 9, wherein the equilibrated adequacy parameter is the eKt/V of absorbance, UV-absorbance, or any uremic compound removed during a kidney substitution treatment.

11. The method and device according to claim 1, wherein the suitable medium to store the modeled physiological parameters is a hard disk hosted in the kidney substitution treatment machine, a memory card hosted in the kidney substitution treatment machine or a patient card attached to the kidney substitution treatment machine.

12. The method and device according to claim 1, wherein the estimated curves describing the adequacy of the kidney substitution treatment arc the timely decay of absorbance, UV-absorbance, or concentration of any uremic substance cleared during a kidney substitution treatment.

13. The method and device according to claim 1, wherein the identification of abnormalities during a kidney substitution treatment is done by defining a critical level; and wherein a difference between the estimated curve describing the adequacy of the kidney substitution treatment and the actual curve describing the adequacy of the kidney substitution treatment greater than the defined level identifies the abnormality.

14. The method and device according to claims 1 and 13, wherein the abnormality is either a clinical or sub-clinical hypotensive episode defined by a bump in the absorbance curve; and/or a dialyzer clotting identified by a steep drop on the absorbance curve together with an increase of the pressure difference between dialyzer inlet and outlet; and/or access recirculation defined by a steep drop on the absorbance curve.

15. The method and device according to claims 1 and 13; wherein a proper action to handle the identified abnormality of the kidney substitution treatment is triggered on the kidney substitution treatment machine.

16. The method and device according to claims 1, 13 and 14, wherein the action triggered by the identification of a deviation is measuring the blood pressure and/or stopping the ultrafiltration pump and/or administering a bolus of saline solution.

17. The method and device according to claims 1, 13 and 14, wherein the action triggered by the identification of a deviation is notifying, warning and/or alarming the medical staff..

18. A kidney substitution treatment machine, wherein the method according to any one of claims 1 to 17 is implemented.

EP 2 292 283 A1

figure 1

figure 2

20

figure 3

figure 4

figure 5

figure 6

figure 7

EP 2 292 283 A1

**EUROPEAN SEARCH REPORT**

Application Number

EP 09 01 1352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 98/55166 A1 (GAMBRO AB [SE]; STERNBY JAN [SE]) 10 December 1998 (1998-12-10)<br>* abstract; claims 22-42; figure 1 *<br>* page 1, lines 11-14 *<br>* page 3, line 11 - page 4, line 27 *<br>* page 6, line 8 - page 7, line 33 *<br>* page 8, line 30 - page 9, line 11 *<br>* page 9, line 12 - page 10, line 2 *<br>* page 16, line 28 - page 17, line 2 *<br>* page 18, lines 13-30 *<br>* page 19, lines 15-25 * | 1-3,5,<br>7-17<br>4,6 | INV.<br>A61M1/16<br>A61B10/00 |
| X<br>Y | US 5 662 806 A (KESHAVIAH PRAKASH [US] ET AL) 2 September 1997 (1997-09-02)<br>* abstract; claims 1-6 *<br>* column 3, line 65 - column 4, line 18 *<br>* column 4, line 49 - column 5, line 25 *<br>* column 6, line 46 - column 8, line 67; figures 3,4 * | 1-3,5-6,<br>10-18<br>4,6 | |
| X<br>Y | EP 2 005 982 A1 (BRAUN AVITUM AG B [DE]) 24 December 2008 (2008-12-24)<br>* abstract; claims 11-13; figure 1 *<br>* paragraphs [0001], [0017] - [0019], [0022], [0043] * | 18<br>4 | TECHNICAL FIELDS SEARCHED (IPC)<br><br>A61B<br>A61M |
| X,D<br>Y | WO 99/62574 A1 (ALTHIN MEDICAL AB [SE]; FALKVALL THORE [SE]; SANDBERG LARS OLOF [SE];) 9 December 1999 (1999-12-09)<br>* abstract; figure 1 *<br>* page 4, line 31 - page 6, line 18 *<br>* page 7, lines 10-17; claims 1,2,4-6,8 * | 18<br>4 | |

-/--

The present search report has been drawn up for all claims

| Place of search | Date of completion of the search | Examiner |
|---|---|---|
| Munich | 4 February 2010 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

24

| | | Europäisches Patentamt European Patent Office Office européen des brevets | **EUROPEAN SEARCH REPORT** | Application Number EP 09 01 1352 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/23311 A1 (GAMBRO AB [SE]; SHALDON STANLEY [FR]; GRANOLLERAS CELINE [FR]) 4 June 1998 (1998-06-04) * abstract; claims 11-14 * * page 1, lines 12-15 * * page 1, line 35 - page 2, line 9 * * page 7, lines 21-24 * * page 8, lines 18-23 * * page 8, line 29 - page 9, line 25 * | 18 | |

TECHNICAL FIELDS SEARCHED (IPC)

The present search report has been drawn up for all claims

| Place of search | Date of completion of the search | Examiner |
|---|---|---|
| Munich | 4 February 2010 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 09 01 1352

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2010

| Patent document cited in search report | | Publication date | Patent family member(s) | | Publication date |
|---|---|---|---|---|---|
| WO 9855166 | A1 | 10-12-1998 | AT | 321580 T | 15-04-2006 |
| | | | AU | 732784 B2 | 26-04-2001 |
| | | | AU | 8046698 A | 21-12-1998 |
| | | | BR | 9809718 A | 11-07-2000 |
| | | | CA | 2292717 A1 | 10-12-1998 |
| | | | DE | 69834034 T2 | 17-08-2006 |
| | | | EP | 0986410 A1 | 22-03-2000 |
| | | | ES | 2260838 T3 | 01-11-2006 |
| | | | JP | 4148536 B2 | 10-09-2008 |
| | | | JP | 2002514120 T | 14-05-2002 |
| | | | US | 6258027 B1 | 10-07-2001 |
| US 5662806 | A | 02-09-1997 | US | 5849179 A | 15-12-1998 |
| EP 2005982 | A1 | 24-12-2008 | WO | 2009013575 A1 | 29-01-2009 |
| WO 9962574 | A1 | 09-12-1999 | AT | 262932 T | 15-04-2004 |
| | | | DE | 69916053 D1 | 06-05-2004 |
| | | | DE | 69916053 T2 | 03-03-2005 |
| | | | EP | 1083948 A1 | 21-03-2001 |
| | | | JP | 2002516722 T | 11-06-2002 |
| | | | SE | 525639 C2 | 22-03-2005 |
| | | | SE | 9801983 A | 05-12-1999 |
| | | | US | 6666840 B1 | 23-12-2003 |
| WO 9823311 | A1 | 04-06-1998 | AT | 298594 T | 15-07-2005 |
| | | | AU | 5141598 A | 22-06-1998 |
| | | | DE | 69733657 D1 | 04-08-2005 |
| | | | DE | 69733657 T2 | 15-12-2005 |
| | | | EP | 0942759 A1 | 22-09-1999 |
| | | | ES | 2245466 T3 | 01-01-2006 |
| | | | JP | 4059926 B2 | 12-03-2008 |
| | | | JP | 2001504735 T | 10-04-2001 |
| | | | US | 6284141 B1 | 04-09-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 183948 B1
- EP 0847768 B1

### Non-patent literature cited in the description

- Replacement of renal function by dialysis. Kluwer academic publishers, 2004, 73-91
- Replacement of renal function by dialysis. Kluwer academic publishers, 2004, 414-418
- **I. Fridolin ; M. Magnusson ; L.-G. Lindberg.** On-line monitoring of solutes in dialysate using absorption of ultraviolet radiation: Technique description. *The International Journal of Artificial Organs,* 2002, vol. 25 (8), 748-761
- **Dedrick RL ; Gabelnick HL ; Bischoff BK.** Kineties of Urea Distribution. *Proc Ann Conf Engr Med Biol,* 1968, vol. 10, 36-41
- **Daugirdas JT.** Formal analytical solution to a regional blood flow and diffusion based urea kinetic model. *ASAIO J.,* July 1994, vol. 40 (3), M667-73
- *ASAIO J.,* July 1994, vol. 40 (3), M667-73